(19) Europäisches Patentamt
European Patent Office
Office européen des brevets

(11) Veröffentlichungsnummer: **0 355 189 B1**

(12) **EUROPÄISCHE PATENTSCHRIFT**

(45) Veröffentlichungstag der Patentschrift: **08.07.92**

(51) Int. Cl.5: **C02F 3/12**, C02F 11/12, C07D 475/04

(21) Anmeldenummer: **88113720.2**

(22) Anmeldetag: **23.08.88**

# (54) Verfahren zum Eindicken/Entwässern von Klärschlamm.

(43) Veröffentlichungstag der Anmeldung:
**28.02.90 Patentblatt 90/09**

(45) Bekanntmachung des Hinweises auf die Patenterteilung:
**08.07.92 Patentblatt 92/28**

(84) Benannte Vertragsstaaten:
**BE DE FR GB IT**

(56) Entgegenhaltungen:

**CHEMICAL ABSTRACTS, Band 104, Nr. 12, 24. März 1986, Seite 401, Zusammenfassung Nr. 94866c, Columbus, Ohio, US;**

(73) Patentinhaber: **Blum, Holger**
**Parkallee 75**
**W-2000 Hamburg 13(DE)**

(72) Erfinder: **Blum, Holger**
**Parkallee 75**
**W-2000 Hamburg 13(DE)**

(74) Vertreter: **Patentanwälte Kirschner & Grosse**
**Forstenrieder Allee 59**
**W-8000 München 71(DE)**

**EP 0 355 189 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft ein neues Verfahren zum Eindicken und Entwässern von Klärschlamm, welcher Anteile an Belebtschlamm aus einer belüfteten biologischen Reinigungsstufe enthält.

Das Verfahrensziel bei der industriellen und/oder kommunalen biologisch- aeroben Abwasserreinigung ist die Erzeugung eines leicht filtrierbaren, kompakten Klärschlammes mit hohen Trockenstoffgehalt im Filterkuchen. Die mit einem hohen Trockenstoffgehalt einhergehende Verringerung des Schlammvolumens durch Abtrennung des Wasseranteils ermöglicht eine kostengünstige und energiesparende Weiterverwendung des Klärschlammes durch Lagerung auf Deponien, Verbrennung, Ausfaulung oder Verwendung in der Landwirtschaft.
Klärschlamm besteht aus dem in der Vorklärung durch Fällung in Gegenwart von organischen und/oder anorganischen Flockungshilfsmitteln gewonnenen sog. Primärschlamm und aus dem in der biologischen Reinigungsstufe erzeugten Belebtschlamm. Je nach der Art und Betriebsweise der Kläranlage enthält der Klärschlamm zwischen ca. 10 % bis 90 Gew% Belebtschlamm. Während der Primärschlamm allein, entsprechend seiner Herkunft durch Fällung im Vorklärbecken im allgemeinen gute Filtrierbarkeitswerte (Trockenstoffanteil und Filtriergeschwindigkeit) aufweist, sind Mischungen aus Primärschlamm und Belebtschlamm im allgemeinen schwierig einzudicken und zu entwässern.

Nach dem Stand der Technik wird dem aus der biologischen Reinigungsstufe stammenden Belebtschlamm, dem sog. Überschußschlamm im Nachklärbecken zur Verbesserung des Sedimentations- und Entwässerungsverhalten ein sog. Flockungshilfsmittel zugegeben. Der so vorbehandelte Überschußschlamm wird im Nacheindicker mit dem Primärschlamm gemischt und das Schlammgemisch in Zentrifugen, Band- und Kammerfilterpressen entwässert.

Die Zugabe der Flockungshilfsmittel kann statt im Nachklärbecken auch an einer anderen Stelle, beispielsweise in der sog. Nacheindickung - eindosiert in die Mischung aus Primär- und Überschußschlamm - erfolgen. Stets enthält der Filter- oder Zentrifugenkuchen des Klärschlammes außer Primär- und Belebtschlamm mehr oder weniger große Anteile an organischen und/oder anorganischem Flockungshilfsmittel.
Die Zusammensetzung und die Anwendungsweise der Flockungshilfsmittel ist dem Fachmann bekannt.
Bei einer vielverwendeten Methode wird der zu filtrierende Schlamm mit Eisen-III-salz, z.B. Einsen-III-chlorid und Calciumhydroxid behandelt, derart, daß ca. 20 bis 60 Kilogramm Eisen-III-chlorid und 75 bis 180 Kilogramm Calcium Hydroxid pro Tonne trockener Feststoff angewendet werden.
Man erhält durch diese Verfahrensweise einen kompakten leicht filtrierbaren Klärschlamm, der allerdings durch zusätzliche Mengen an schlackenbildenden anorganischen Stoffen belastet ist.
Neben dieser sog. Eisen-Kalkmethode werden vorzugsweise organische polymere Flockungsmittel zur Eindickung/Entwässerung von Belebtschlamm enthaltendem Klärschlamm eingesetzt. Polymere Flockungsmittel stellen wasserlösliche, kettenförmige Polymerisate dar, die meist durch Polymerisation von Acrylamid hergestellt werden (z.B. nach DOS 2025 725 oder DOS 2337 337 oder Japan Kokai 75161 479 oder Japan Kokai 75 53 274 oder Japan Kokai 74 59855). Es lassen sich aber auch andere polymerisierbare Monomer, z.B. Ethylenoxid (UDSSR Pat.SU 1204576), Ethylenimin, Allylguanidine (USA Pat. 3878 170) oder quaternäre Carbonsäureester (Japan Kokai 7604084) oder Polyamine (USA Pat 3174928, Span.Pat 287 939, Japan Kokai Tokio Koho 86192 734) zu kettenförmigen Polyelektrolyten vereinigen. Je nach ihrer elektrostatischen Ladung in wässriger Lösung unterscheidet man zwischen nichtionogenen, anionenaktiven und kationenaktiven polymeren Flockungsmitteln. Auch die Kombination anorganisch/organisch, wie Braunkohlenstaub plus Polymer, ist als Flockungsmittel dem Fachmann bekannt.
Je Tonne Feststoff werden etwa 1 Kilogramm bis 7 Kilogramm polymerer Polyelektrolyt benötigt, um eine ausreichend schnelle Eindickung und Entwässerung des Klärschlammes zu erreichen.
Durch die Verwendung von organischen Polyelektrolyten wird das Problem der großen Menge an schlakkenbildenden anorganischen Stoffen im Filterkuchen des Klärschlammes (Eisen/Kalkmethode) zwar gelöst, dafür stellen die statt dessen eingesetzten organischen Flockungsmittel einen beachtlichen Kostenfaktor dar, der die Wirtschaftlichkeit der Schlammeindickung und Entwässerung entscheidend mindern kann.
Es hat daher nicht an Versuchen gefehlt, die Verfahrensweise der Schlammeindickung und Entwässerung mittels der vorgenannten organischen polymeren Flockungsmittel so zu modifizieren, daß deren notwendige Einsatzmenge auf ein Mindestmaß reduziert wird.

Beispielsweise lehrt Patentschrift Japan Kokai Tokio Koho 79113954 die Verwendung von kationenaktiven nichtionogenen Copolymerisaten von bestimmten molaren Verhältnissen zur Leistungssteigerung bei der Entwässerung von Schlämmen. Europ. Pat Appl. EP 159 178 sowie Japan Kokai Tokio Koho 87102 892 lehren die Verwendung von synergistisch wirkenden anorganischen/organischen Flockungs/Beschwerungsmitteln zwecks Einsparung von Polymeren. In der Patentschrift Japan Kokai 75141850 wird eine Verfahrensweise für die Eindickung/Entwässerung von Klärschlämmen mit vermindertem Polymerverbrauch beschrieben, in welcher der Schlamm zunächst mit einem Teil der abgesetzten Kläre - einer vorherigen Fällung - behandelt wird, wonach unter Verwendung einer verminderten Menge an frischem Polymer die Eindickung des vorbehandelten Schlammes erfolgt. Japan Kokai 75 86 476 beschreibt für die Schlammentwässerung eine synergistisch wirkende Kombination aus Aluminiumsalzen und nichtionogenem Polyacrylamid.

Die vorstehend beschriebenen synergistischen Verfahrensweisen setzen eine genaue Überwachung der Eigenschaften des zu entwässernden Schlammes sowie eine ständige analytische Kontrolle des Fällungs/Entwässerungsvorganges voraus, um die in den Patentschriften angegebenen Vorteile nutzen zu können. Beispielsweise beschreibt Japan Kokai Tokio Koho 87132 600 den Analysenaufwand zur Vorhersage des Entwässerungsverhaltens des Klärschlammes. Bei den in der kommunalen und industriellen Praxis der Klärschlammaufbereitung vorkommenden großen Schwankung des Flockungs- und Entwässerungsverhaltens des Mischschlammes sind der Anwendung der obenerwähnten synergistischen Verfahrensweisen Grenzen gesetzt.

Aufgabe der Erfindung war es daher, eine Verfahrensweise zur Eindickung/Entwässerung von Klärschlamm zu finden, welche unabhängig von Schwankungen des Flockungs- und Entwässerungsverhaltens von Belebtschlamm enthaltendem Klärschlamm eine wirkliche Reduktion der notwendigen Menge an organischem und/oder anorganischem Flockungshilfsmittel ermöglicht.

Chem. Abstracts, Bd. 104, Nr. 12, Seite 401, Nr. 94 866 C beschreibt ein Verfahren, bei dem der Belebtschlammanteil in einer belüfteten biologischen Reinigungsstufe in Gegenwart von Riboflavin, Thiamin, Folsäure und Panthothensäure erzeugt wird. Bei diesem Verfahren wird der Schlamm auch entwässert, wobei nicht explizit offenbart ist, daß Flockungsmittel zugesetzt werden. Der Einfluß der Zusätze auf die Entwässerbarkeit des Klärschlamms bzw. die Reduzierung des Bedarfs an Flockungsmitteln ist nicht angesprochen.

Es wurde nun gefunden, daß diese Aufgabe erfindungsgemäß dadurch gelöst werden kann, daß der Belebtschlammanteil des zu entwässernden Klärschlammes in einer belüfteten biologischen Reinigungsstufe in Gegenwart von 1 bis 0,01 Gewichtsteile pro Million Gewichtsteile (ppm)- bezogen auf die der biologischen Reinigungsstufe zufließende Flüssigkeitsmenge - von Folsäure und/oder Dihydrofolsäure und/oder mindestens einem Salz derselben erzeugt wird.

Gegenstand der Erfindung ist eine Verfahrensweise zum Eindikken/Entwässern von Belebtschlamm enthaltendem Klärschlamm mit verringertem Bedarf an anorganischem/organischem Flockungsmitteln, die dadurch gekennzeichnet ist, daß der Belebtschlammanteil in einer belüfteten biologischen Reinigungsstufe erzeugt wird in Gegenwart von Folsäure und/oder Dihydrofolsäure und/oder mindestens einem von deren Salzen erzeugt wird, mit der maßgabe, daß das Verfahren mit Folsäure ohne Zugabe von Riboflavin, Thiamin und Panthothensäure durchgeführt wird.

Die erfindungsgemäße Verfahrensweise gestattet es, im wesentlichen unabhängig von Schwankungen in der Zusammensetzung und dem Entwässerungsverhalten des Primärschlammes eine gleichmäßige Betriebsweise der Schlammeindickung und Filtration/ Zentrifugierung durchzuführen und erfordert nur einen minimalen Aufwand bei der analytischen Überwachung der Verfahrensschritte. Darüberhinaus gestattet es die erfindungsgemäße Verfahrensweise, im Gegensatz zu früher bekannten Verfahren, eine bedeutende Reduktion an Flockungsmitteln vorzunehmen, ohne daß neue umweltschädigende Stoffe in den Kreislauf der industriellen oder kommunalen Abwasseraufbereitung eingeführt werden.

Die Folsäure und/oder Dihydrofolsäure und/oder deren Salze werden in Mengen von 5 bis 0,01 ppm, vorzugsweise 1 bis 0,01 ppm, bezogen auf die der biologischen Reinigungsstufe zufließendem Abwasser, zugesetzt.

Gemäß einer bevorzugten Ausgestaltung der Erfindung wird dem der biologischen Reinigungsstufe zufließenden vorgeklärtem Abwasser ständig etwa 1 bis 0,01 ppm an Folsäure und/oder Dihydrofolsäure und/oder mindestens einem Ammonim-Alkalimetall-, Erdalkalimetall- und/oder Alkanolammoniumsalz derselben zudosiert. Nach einigen Tagen kann dann die bisher in der Klärschlammeindickung/Entwässerung notwendige Menge an Flockungsmitteln stufenweise zurückgenommen werden, ohne daß sich das Absetz- und Entwässerungsverhalten des Klärschlammes verschlechtert.

Bei einer anderen erfindungsgemäßen Verfahrensweise wird, bezogen auf das in die biologische Reinigungsstufe zufließende, vorgeklärte Abwasser ständig 1 bis 0,01 ppm an Folsäure und/oder Dihydrofolsäure und/oder mindestens einem Ammonium-Alkalimetall-, Erdalkalimetall- und/oder Alkanolammoniumsalz derselben in den sog. Schlammrücklauf zudosiert. Nach einigen Tagen kann dann die zuvor in der Klärschlammeindickung/ Entwässerung notwendige Menge an Flockungsmitteln stufenweise zurückgenommen werden, ohne daß sich das Absetz- und Entwässerungsverhalten des Klärschlammes verschlechtert.

Da es sich bei Folsäure und/oder Dihydrofolsäure und/oder deren Salzen um langsamlösliche feste Stoffe handelt, werden diese vorgenannten Stoffe und deren Salze bevorzugt verdünnt als wässrige Lösung

in das Belebtschlammbecken zudosiert. Bei Zugabe der Folsäure/Dihydrofolsäure und/oder deren Salze in fester Form zum Belebtschlamm konnte nur bei starker Überdosierung eine geringe Verbesserung des Entwässerungs-Absetzverhaltens des resultierenden Mischschlammes erzielt werden.

Wird das erfindungsgemäße Verfahren durchgeführt, indem man zum Belebtschlamm in der aeroben biologischen Reinigungsstufe wässrige Alkali- oder Erdakalisalzlösungen oder Alkanolammoniumsalze der Folsäure

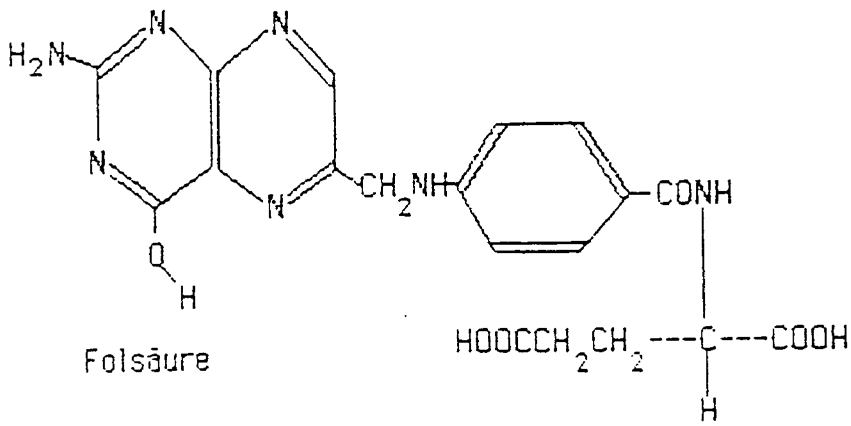

Folsäure

zudosiert und den so behandelten Überschußschlamm allein oder mit Primärschlamm gemischt entwässert; so ist infolge der mangelnden Stabilität der Folsäure in wässriger Lösung eine Konzentration von ca. 0,5 bis 3 ppm Folsäure bezogen auf das dem Belebtbecken zufließende Abwasser erforderlich, um den gewünschten Verbesserungseffekt bei der Entwässerung des Klärschlammes zu erzielen.
Wird demgegenüber, statt reiner Folsäure deren Derivat Dihydrofolsäure

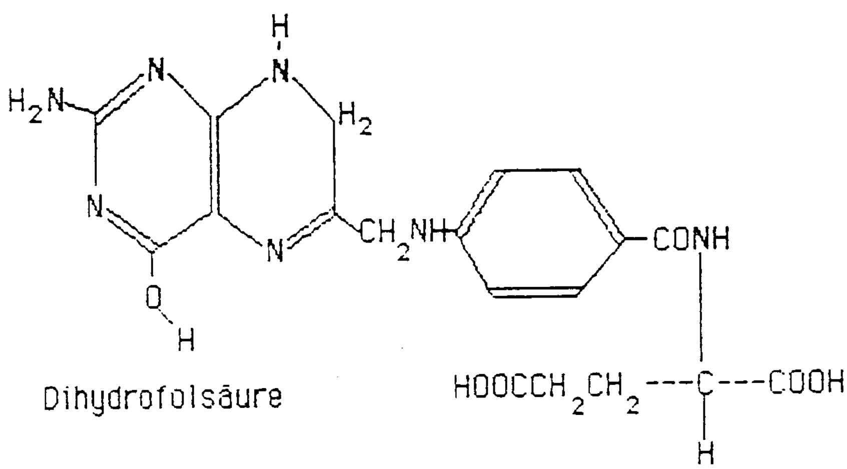

Dihydrofolsäure

und/oder Mischungen von Folsäure und Dihydrofolsäure als Alkali- oder Erdalkalisalzlösungen oder Alkanolammoniumsalzlösungen zum Belebtschlamm in der aeroben biologischen Reinigungsstufe zudosiert, so ist infolge der guten Stabilität der Folsäure/Dihydrofolsäure-Mischung in wässriger Lösung nur eine Konzentration von ca. 0,01 bis 0,1 ppm Folsäure/Dihydrofolsäure-Mischung bezogen auf das dem Belebtbecken zufließende Abwasser erforderlich, um den gewünschten Verbesserungseffekt bei der Entwässerung des Klärschlammes zu erzielen.

Stabile Folsäurezubereitungen sind in der EP-A-87 118 082.4 beschrieben.

Unter den obengenannten Alkalimetallsalzen sind die Lithium-, Natrium-, Kalium-, Rubidium- und Caesiumsalze zu verstehen.

Unter dem hier verwendeten Ausdruck "Erdalkalimetallsalze" sind die Magnesium-, Calcium-, Strontium- und Bariumsalze zu verstehen.

Unter dem obengenannten Ausdruck "Ammoniumsalze" sind sowohl die Ammoniumsalze als auch die

Tetraalkylammoniumsalze mit dem Kation NH$\hat{4}$ bzw. NR$\hat{4}$ zu verstehen, worin R einen niederen Alkylrest mit vorzugsweise 1 bis 6, insbesondere 1 bis 4, speziell 1 bis 3 Kohlenstoffatomen, bedeutet.

Bei dem Alkanolammoniumsalz handelt es sich vorzugsweise um ein Salze der obengenannten organischen Säuren mit einem Dialkanolamin der Formel

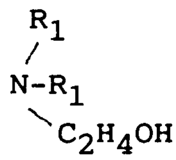

Dabei bedeutet R1 Wasserstoff und/oder Hydroxyäthyl und/oder Hydroxypropyl.

Ein Kennzeichen des erfindungsgemäßen Verfahrens ist die Tatsache, daß der Effekt der verbesserten Schlammentwässerung zeitverzögert auftritt. Im allgemeinen sind 5 bis 10 Tage Vorlauf in der Zugabe von Folsäure/Dihydrofolsäurelösung erforderlich bevor der Effekt einsetzt. Wird die Zugabe abgebrochen, so kann der Effekt noch einige Tage danach beobachtet werden.

Obzwar bekannt ist, daß Folsäure in Kläranlagen kaum vorhanden ist und das Wachstum verschiedener Mikroorganismen stimuliert (MOHR, H.; Folsäure - Mikronährstoff und Wachstumsförderer für Bakterien und Pilze. Eine Übersicht, BioTechnologie 10, 1987) war nicht zu erwarten, daß sich beim Zusatz der vorgenannten Folsäure und/oder Dihydrofolsäure und/oder ihrer Salze zur biologischen aeroben Reinigungsstufe bei der Entwässerung von Mischschlamm eine derartige synergistische Wirkung mit den gebräuchlichen Flockungsmitteln ergibt.

Die erfindungsgemäße Verfahrensweise wird durch die folgenden Beispiele näher erläutert, ohne jedoch darauf beschränkt zu sein.

Beispiel 1

In einer kommunalen Kläranlage wurde eine Mischung aus 30 - 40 Gew% Belebtschlamm und 70-60 Gew% Primärschlamm in einer Kammerfilterpressenanlage verarbeitet. Der im Stapelbehälter vor der Filterpresse befindliche Mischschlamm enthielt zwecks Erhöhung der Filterfähigkeit ca. 4 Gew% Eisen-III-chlorid und 8 Gew% Calciumhydroxid - bezogen auf die Schlammtrockensubstanz. Mit dieser Menge an Flockungsmitteln wurde eine Filterleistung zwischen 25 und 40 Kilogramm /m2/Bar/Stunde erreicht. Der Gehalt an Trockenstoff-Klärschlamm in Filterkuchen lag bei 26/28 Gew%. Diese Verfahrensweise und Betriebswerte hatten sich im Zeitraum von 6 Monaten vor Umstellung auf die erfindungsgemäße Verfahrensweise als konstant und optimal erwiesen.

Zur Umstellung auf die erfindungsgemäße Verfahrensweise wurde im Zulauf zum Belebtbecken eine 0,5-%ige Lösung des Dinatriumsalzes der Folsäure in destilliertem Wasser mittels einer Tropfeinrichtung dosiert. Durch das Belebtbecken flossen täglich etwa 2500 cbm Abwasser aus der Vorklärung. Die Schlammkonzentration im Belebtbecken lag zwischen 2 und 4 Gramm Trockenstoff/Liter, der chemische Sauerstoffbedarf im Vorklärbecken lag zwischen 700 und 1100 mg/Liter. Diese Betriebswerte entsprachen denen der Vorperiode. Die Dosiervorrichtung für die wässrige Folsäurelösung war so eingestellt, daß zu jedem in das Belebtbecken einfließenden Kubikmeter vorgeklärten Abwassers 1,1 Gramm Natriumfolat zudosiert wurde (= Folsäurekonzentration 1 ppm).

Zehn Tage nach Beginn der Folatdosierung war die Filterleistung auf 50-60 Kilogramm /m2/Bar/Stunde gestiegen. Die Eisen/Kalk-Menge wurde daraufhin auf ca. 3 Gew% Eisen-III-chlorid und 4 Gew% Calciumhydroxid bezogen auf die Schlammtrockensubstanz reduziert, ohne daß sich eine Verschlechterung der Filterleistung ergab. Nach weiteren 10 Tagen der Folatdosierung konnte die Eisen/Kalk-Menge auf ca. 2 Gew% Eisen-III-chlorid und 3 Gew% Calcimhydroxid - bezogen auf die Schlammtrockensubstanz - reduziert werden, wobei die Filterleistung bei 50-60 Kilogramm /m2/Bar/Stunde konstant blieb.
Nach 40 Tagen wurde die Folatdosierung unterbrochen, danach blieb die gute Filterleistung bis zum 48. Tage erhalten und verschlechterte sich dann zusehends. Nach 60 Tagen war wieder eine Flockungsmittelmenge von ca. 4 Gew% Eisen-III-chlorid und 8 Gew% Calciumhydroxid - bezogen auf die Schlammtrockensubstanz - erforderlich, um eine ausreichende Filterleistung zu erzielen.

Beispiel 2 (Vergleichsbeispiel)

Der Versuch von Beispiel 1 wurde auf der gleichen Kläranlage wiederholt, indem zu jedem in das Belebtbecken einfließenden Kubikmeter vorgeklärten Abwassers 0,11 Gramm Natriumfolat in einem Liter Wasser gelöst zudosiert wurde. (= Folsäurekonzentration 0,1 ppm). Eine verbesserte Filtratleistung und damit eine Verringerung der Eisen/Kalk-Menge wurde nicht erreicht.

Beispiel 3

In einer Mineralölraffinerie wurde mittels vier hintereinandergeschalteten Belebungsbecken eine tägliche Fracht mit einem biologischen Sauerstoffbedarf von 200 kg BSB5/d (BSB5 = biologischer Sauerstoffbedarf in 5 Tagen) abgebaut. Der belebte Schlamm gelangte in ein Nachklärbecken, wo der Überschußschlamm abgezogen und in ein Schlammstapelbecken gefördert wurde. Der Überschußschlamm wurde auf drei baugleichen Kammerfilterpressen unter Zuhilfenahme von 20 Kiloramm Kalkhydrat und 8 Kilogramm Eisen-III-chlorid je Kubikmeter Filterkuchen auf der Filterpresse entwässert, zum zu einem deponierbaren Filterkuchen mit etwa 35% Feststoffgehalt zu gelangen.
Zur Umstellung auf die erfindungsgemäße Verfahrensweise wurde in das zweite der hintereinandergeschalteten Belebungsbecken zu jedem einfließenden Kubikmeter Abwasser 0,23 Gramm technisches 90-%iges Calcium-dihydrofolat-dihydrat - vorgelöst in 1 Kilogramm destilliertem Wasser - zudosiert (= Folsäurekonzentration 0,018 ppm, Dihydrofolsäurekonzentration = 0,18 ppm). Nach 30 Betriebstagen der erfindungsgemäßen Verfahrensweise hatte sich die Filtrationsgeschwindigkeit des Klärschlammes (Belebtschlammanteil 70 Gew%) so erhöht, daß, bei gleichbleibender Feststoffkonzentration, statt drei nur noch zwei der vorhandenen Kammerfilterpressen benötigt wurden, um die anfallende Schlammenge zu entwässern. Gleichzeitig konnte der Bedarf an Flockungsmitteln auf 10 Kilogramm Kalkhydrat und 4 Kilogramm Eisen-III-chlorid je Kubikmeter Filterkuchen reduziert werden.

Beispiel 4

Es wurde eine wässrige Lösung von Folsäure, Dihydrofolsäure und Citronensäure unter Verwendung von Natronlauge und Kalilauge hergestellt. Der pH-Wert der Lösung betrug 10,4. 100 g Lösung enthielten 8 mMol Folsäuresalz, 8 mMol Dihydrofolsäuresalz und 5 mMol Citronensäuresalz.
Die vorstehende Folsäure/Dihydrofolsäurezubereitung wurde - nachdem sie weiter mit Leitungswasser im Verhältnis 1 : 100 Volumenteile verdünnt worden war, in die Belebtschlammrücklaufleitung der belüfteten Reinigungsstufe eines kommunalen Abwasserzweckverbandes dosiert. Die Kläranlage enthielt keinen Faulturm, die Entwässerung des Klärschlammes geschah mit Hilfe eines hochmolekularen, wasserlöslichen, kationischen Polymers auf Basis von Acrylsäureestern. Täglich wurden in die vier mit Tauchkerzenbelüftung ausgestatteten, parallelen Belebtbecken dieser Anlage 60.000 Kubikmeter Abwasser aus der Vorklärung eingeleitet. Berücksichtigt wurde die Tatsache, daß ein Teil der dosierten Folsäure/Dihydrofolsäurezubereitung verlorenging, da nur 70 % des Rücklaufschlammes in die Belebtbekken zurückgeführt wurde. Es ergab sich somit eine effektive Dosierkonzentration von 0,01 ppm Folsäure und 0,01 ppm Dihydrofolsäure in jedem den parallelen Belebtbecken zufließenden Kubikmeter Abwasser.
Nach 15 Tagen Betriebsdauer mit der erfindungsgemäßen Verfahrensweise konnte, unter Aufrechterhaltung der gleichbleibenden Folatdosierung, die Menge des bei der Entwässerung des Klärschlammes eingesetzten kationischen Polymers auf 30 % desjenigen Wertes reduziert werden, der vor der Anwendung der Folsäure/Dihydrofolsäurezubereitung notwendig war.

## Patentansprüche

1. Verfahren zum Eindicken/Entwässern von Belebtschlamm enthaltendem Klärschlamm mit Hilfe von organischen Polyelektrolyten und/oder anorganischen Flockungsmitteln, **dadurch gekennzeichnet,** daß der Belebtschlammanteil in einer belüfteten biologischen Reinigungsstufe in Gegenwart von Folsäure und/oder Dihydrofolsäure und/oder mindestens einem Salz davon erzeugt wird, mit der Maßgabe, daß das Verfahren mit Folsäure ohne Zugabe von Riboflavin, Thiamin und Panthothensäure durchgeführt wird.

2. Verfahren nach Anspruch 1, **dadurch gekennzeichnet,** daß der Belebtschlammanteil in Gegenwart von 5 bis 0,01 Gewichtsteilen pro Million Gewichtsteile (ppm), bezogen auf die der biologischen Reinigungsstufe zufließende Flüssigkeitsmenge, Folsäure und/oder Dihydrofolsäure und/oder mindestens einem Salz davon erzeugt wird.

3. Verfahren nach Anspruch 2, **dadurch gekennzeichnet,** daß der Belebtschlammanteil in Gegenwart von 1 bis 0,01 Gewichtsteile pro Million Gewichtsteile (ppm), bezogen auf die der biologischen Reinigungsstufe zufließende Flüssigkeitsmenge, Folsäure und/oder Dihydrofolsäure und/oder mindestens einem Salz davon erzeugt wird.

4. Verfahren nach Anspruch 2 oder 3, **dadurch gekennzeichnet,** daß die Folsäure und/oder Dihydrofolsäure und/oder mindestens ein Salz davon dem der biologischen Reinigungsstufe zufließenden vorgeklärten Abwasser ständig zudosiert wird.

5. Verfahren nach Anspruch 2 oder 3, **dadurch gekennzeichnet,** daß die Folsäure und/oder Dihydrofolsäure und/oder mindestens ein Salz davon dem Schlammrücklauf ständig zudosiert wird.

6. Verfahren nach einem der Ansprüche 1 bis 5, **dadurch gekennzeichnet,** daß als Salze die Alkalimetall-, Erdalkalimetall-, Ammonium- und/oder Alkanolammoniumsalze der Folsäure und/oder Dihydrofolsäure eingesetzt werden.

7. Verfahren nach Anspruch 6, **dadurch gekennzeichnet,** daß die Natrium-, Kalium-, Calcium- und/oder Hydroxyethylammoniumsalze der Folsäure und/oder Dihydrofolsäure eingesetzt werden.

8. Verfahren nach einem der Ansprüche 1 bis 7, **dadurch gekennzeichnet,** daß die Folsäure und/oder Dihydrofolsäure und/oder mindestens ein Salz davon in wässriger Lösung zugesetzt werden.

9. Verwendung von Folsäure und/oder Dihydrofolsäure und/oder mindestens einem Salz davon zur Erzeugung von gut eindickbarem/entwässerbarem Belebtschlamm enthaltenem Klärschlamm in Gegenwart von organischen Polyelectrolyten und/oder anorganischen Flockungsmitteln.

**Claims**

1. Method for concentrating/dehydrating sewage sludge including activated sludge with the aid of organic polyelectrolytes and/or inorganic flocculators, **characterized in that** that the activated sludge portion is produced in an aerated biologic clarification step in presence of folic acid and/or dihydrofolic acid and/or at least one salt thereof, provided that the method is carried out with folic acid without adding riboflavine, thiamine and pantothenic acid.

2. Method as defined in claim 1, **characterized in that** the activated sludge portion is produced in the presence of 5 to 0.01 parts by weight per million parts by weight (ppm), based on the amount of liquid flowing into the biologic clarification step, folic acid and or dihydrofolic acid and/or at least one salt thereof.

3. Method as defined in claim 2, **characterized in that** the activated sludge portion is produced in the presence of 1 to 0.01 parts by weight per million parts by weight (ppm), based on the amount of liquid flowing into the biologic clarification step, folic acid and/or dihydrofolic acid and/or at least one salt thereof.

4. Method as defined in claim 2 or 3, **characterized in that** the folic acid and/or dihydrofolic acid and/or at least one salt thereof is continuously dosingly added to presedimented sewage water flowing into the biologic clarification step.

5. Method as defined in claim 2 or 3, **characterized in that** the folic acid and/or dihydrofolic acid and/or at least one salt thereof is continuously dosingly added to sludge back flow.

6. Method as defined in one of claims 1 to 5, **characterized in that** as salts alkali metal salts, alkaline earth metal salts, ammonium salt and/or alkanolammonium salts of the folic acid and/or dihydrofolic acid are used.

7. Method as defined in claim 6, **characterized in that** the sodium, potassium, calcium or hydroxyethyl ammonium salts of the folic acid and/or dihydrofolic acid are used.

8. Method as defined in one of claims 1 to 7, **characterized in that** the folic acid and/or dihydrofolic acid and/or at least on salt thereof is added in aqueous solution.

9. Use of folic acid and/or dihydrofolic acid and/or at least one salt thereof for the production of sewage sludge including well concentratable/dehydratable activated sludge in the presence of organic polyelectrolytes and/or inorganic flocculators.

**Revendications**

1. Procédé pour la concentration/déshydratation de boue d'épuration contenant de la boue activée au moyen de polyélectrolytes organiques et/ou de floculants inorganiques, caractérisé en ce que la fraction de boue activée est produite dans une étape d'épuration biologique aérée en présence d'acide folique et/ou d'acide dihydrofolique et/ou d'au moins un de Leurs sels, avec la condition que le procédé est mis en oeuvre avec l'acide folique sans addition de riboflavine, de thiamine et d'acide pantothénique.

2. Procédé selon la revendication 1, caractérisé en ce que la fraction de boue activée est produite en présence de 5 à 0,01 partie en poids d'acide folique et/ou d'acide dihydrofolique et/ou au moins un de leurs sels par million de parties en poids, par rapport à la quantité de liquide arrivant à l'étape d'épuration biologique.

3. Procédé selon la revendication 2, caractérisé en ce que la fraction de boue activée est produite en présence de 1 à 0,01 partie en poids d'acide folique et/ou d'acide dihydrofolique et/ou au moins un de leurs sels par million de parties en poids, par rapport à la quantité de liquide arrivant à l'étape d'épuration biologique.

4. Procédé selon la revendication 2 ou 3, caractérisé en ce que l'on dose en continu l'acide folique et/ou l'acide dihydrofolique et/ou au moins un de leurs sels dans l'eau d'égout préclarifiée arrivant à l'étape de purification biologique.

5. Procédé selon la revendication 2 ou 3, caractérisé en ce que l'on dose en continu l'acide folique et/ou l'acide dihydrofolique et/ou au moins un de leurs sels dans la boue de recyclage.

6. Procédé selon l'une des revendications 1 à 5, caractérisé en ce que l'on utilise comme sels les sels de métaux alcalins, les sels de métaux alcalino-terreux, les sels d'ammonium et/ou les sels d'alcanolammoniums de l'acide folique et/ou de l'acide dihydrofolique.

7. Procédé selon la revendication 6, caractérisé en ce que l'on utilise les sels de sodium, de potassium, de calcium et/ou d'hydroxyéthylammonium de l'acide folique et/ou de l'acide dihydrofolique.

8. Procédé selon l'une des revendications 1 à 7, caractérisé en ce que l'acide folique et/ou l'acide dihydrofolique et/ou au moins un de leurs sels sont ajoutés en solution aqueuse.

9. Utilisation de l'acide folique et/ou de l'acide dihydrofolique et/ou d'au moins un de leurs sels pour la production de boue d'épuration contenant de la boue activée, facile à épaissir/ déshydrater en présence de polyélectrolytes organiques et/ou de floculants inorganiques.